# EUROPEAN PATENT SPECIFICATION

(11) **EP 4 266 057 B1**
(45) Date of publication and mention of the grant of the patent: **01.04.2026**
(21) Application number: 22169087.8
(22) Date of filing: 20.04.2022
(51) Int. Cl.: G01N 33/569

(54) **KYNURENINE: USEFUL BIOMARKER IN ACUTE COVID-19 AND LONG COVID**
KYNURENIN: NÜTZLICHER BIOMARKER BEI AKUTEM COVID-19 UND LONG COVID
KYNURÉNINE : BIOMARQUEUR UTILE POUR LE COVID-19 AIGU ET LE COVID LONG

(43) Date of publication of application: 25.10.2023
(73) Proprietor: SALION GmbH, 82541 Münsing (DE)
(72) Inventor: Stangl, Manfred J., 82054 Sauerlach (DE); Abendroth, Dietmar, 89275 Thalfingen (DE)
(74) Representative: Schaafhausen, Anne

(56) References cited:
- EP-A1- 3 499 240
- WO-A1-2018/224696
- LAWLER NATHAN G. ET AL: "Systemic Perturbations in Amine and Kynurenine Metabolism Associated with Acute SARS-CoV-2 Infection and Inflammatory Cytokine Responses", vol. 20, no. 5, 16 March 2021 (2021-03-16), pages 2796 - 2811, XP055961461, ISSN: 1535-3893, Retrieved from the Internet <URL:https://pubs.acs.org/doi/pdf/10.1021/acs.jproteome.1c00052> DOI: 10.1021/acs.jproteome.1c00052
- MANGGE HARALD ET AL: "Increased Kynurenine Indicates a Fatal Course of COVID-19", ANTIOXIDANTS, vol. 10, no. 12, 7 December 2021 (2021-12-07), pages 1960, XP055961437, DOI: 10.3390/antiox10121960
- BI XIAOJIE ET AL: "Proteomic and metabolomic profiling of urine uncovers immune responses in patients with COVID-19", vol. 38, no. 3, 18 January 2022 (2022-01-18), US, pages 110271, XP055961450, ISSN: 2211-1247, Retrieved from the Internet <URL:https://ars.els-cdn.com/content/image/1-s2.0-S2211124721017836-mmc8.pdf> DOI: 10.1016/j.celrep.2021.110271
- VERNEROV� ANDREA ET AL: "Chromatographic method for the determination of inflammatory biomarkers and uric acid in human saliva", TALANTA, ELSEVIER, AMSTERDAM, NL, vol. 233, 9 June 2021 (2021-06-09), XP086682822, ISSN: 0039-9140, [retrieved on 20210609], DOI: 10.1016/J.TALANTA.2021.122598
- CIHAN MURAT ET AL: "Kynurenine pathway in Coronavirus disease (COVID-19): Potential role in prognosis", JOURNAL OF CLINICAL LABORATORY, vol. 36, no. 3, 29 January 2022 (2022-01-29), US, pages 1 - 10, XP093114608, ISSN: 0887-8013, Retrieved from the Internet <URL:https://onlinelibrary.wiley.com/doi/full-xml/10.1002/jcla.24257> DOI: 10.1002/jcla.24257

## Description

The present invention relates to an in vitro method for the detection of inflammation caused by an acute COVID-19 infection, long COVID or PIMS, to the use of kynurenine in the detection of inflammation, and to the use of a test kit for performing such a method.

### Background

The link between host defense against pathogens seems to be the initial tissue injury mediated by a large variety of generated pathogen-associated molecular patterns, PAMPs, and by any injurious nonpathogenic factors associated with the generation and appearance of damage-associated molecular patterns, DAMPs.

There is evidence that in the center of tissue injury, regardless of its origin - infectious, toxic, physical or other injurious events - reactive oxygen species, ROS, play a dominant role. Oral gingival epithelium as well as the airway epithelium are predisposed as a sentinel system to detect pathogens and nonpathogenic agents and to initiate a host innate defense response.

The innate immune responses and relevant cell types play a vital role in the clinical symptoms and severity of COVID-19 disease. This assumption is in agreement with the previous studies on severe acute respiratory syndrome coronavirus, SARS-CoV, the closest relative to the causative agent of COVID-19, SARS-CoV-2, which predominantly infects airway and alveolar epithelial cells, vascular endothelial cells, and macrophages. It was shown that SARS-CoV triggers various innate recognition and response pathways.

The short- and long-term sequelae following recovery of the disease suggests that these syndromes lead to an accelerated state of chronic subclinical systemic inflammation often seen in aging, termed inflammaging, resulting in increased and worsening of age-related conditions including frailty even in younger individuals. These findings were clinically related to different organs like brain, heart, vascular system, kidney and muscles.

The prevailing evidence suggests that patients with a severe COVID-19 disease seem to have an overreaction of the innate immune system demonstrating exacerbated levels of inflammation caused by a cytokine storm.

At this early stage, the mechanisms underpinning COVID-19 are still subject to intense scrutiny and the long-term mental health consequences as a result of the disease are unknown. The activated and not successfully downregulated innate immune response together or alone with a cytokine storm event could lead to an increased risk of long COVID or PIMS. The background of these syndromes is represented by an ongoing subclinical inflammation, not easy to detect and probably due to a not well chosen or not successful type of immunosuppressive therapy.

### Kynurenine

Kynurenine is an aromatic, non-proteinogenic amino acid, and a metabolite of the tryptophan metabolism. Inside the tryptophan metabolism, the kynurenine pathway, KP, plays a critical role in generating cellular energy in the form of nicotinamide adenine dinucleotide, NAD+. Because energy requirements are substantially increased during an immune response, the KP is a key regulator of the immune system. This key regulator is of utmost importance especially in the line of first defense in the innate immune activation.

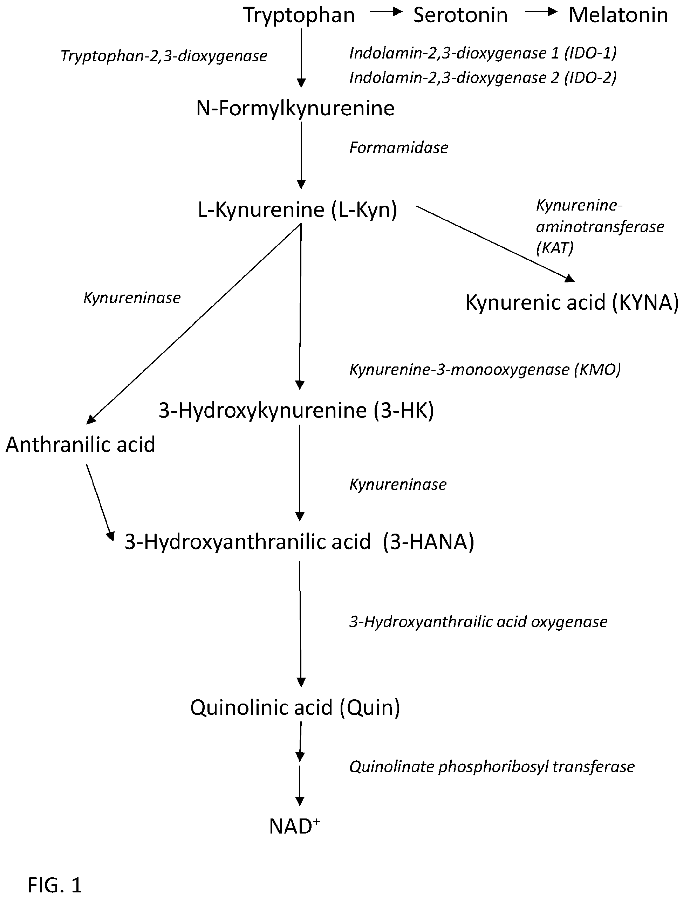

Kynurenine is synthesized by the enzyme tryptophan dioxygenase, which is produced primarily in the liver, and indoleamine-2,3-dioxygenase, IDO-1, which is produced in many tissues in response to immune activation (Figure 1).

### Background to COVID-19

Coronavirus disease 2019 (COVID-19) is a highly infectious disease caused by severe acute respiratory syndrome coronavirus 2, SARS-CoV-2. Common symptoms of COVID-19 include fever, cough, fatigue, shortness of breath, and loss of smell and taste. The majority of cases result in mild symptoms, however, some progress to acute respiratory distress syndrome, ARDS, characterized by a cytokine storm, multi-organ failure, septic shock, and blood clots.

While the mechanism by which only some individuals develop severe respiratory pathology is still not fully clarified, a number of studies have shown that patients who are ill enough to require hospitalization tend to be older persons affected by multimorbidity, including hypertension, diabetes, and/or obesity.

Disease severity in hospitalized patients is characterized by severe pneumonia associated with overt inflammatory reaction characterized by high C-reactive protein, CRP, and interleukin-6, IL-6, low albumin, high sedimentation rate, low eosinophils, and lymphopenia. Hospitalized individuals also have increased lactate dehydrogenase, LDH, a marker of cellular death, often associated with altered coagulation. High LDH, low lymphocyte count and high levels of high-sensitivity CRP predict mortality of individual patients more than 10 days in advance with greater than 90% accuracy.

Thus, an extraordinary proliferation of studies published over the last few months suggests that a SARS-CoV-2 infection unleashes a powerful, and apparently uncontrolled inflammatory response that most likely adds to the tissue damage already caused by the viral infection toward the COVID-19 underlying pathology.

Noteworthy, while severe COVID-19 affects disproportionally older people, systemic inflammation from SARS-CoV-2 infection is detected in patients of all age groups, including a severe multisystem inflammatory syndrome with features of Kawasaki disease identified in children.

### Background to PIMS

Pediatric inflammatory multisystem syndrome, PIMS, is a rare systemic illness involving persistent fever and extreme inflammation following exposure to SARS-CoV-2 in children. The syndrome is also called multisystem inflammatory syndrome in children, MIS-C, or systemic inflammatory syndrome in COVID-19, SISCoV. It can rapidly lead to medical emergencies such as insufficient blood flow around the body, a condition known as shock. Failure of one or more organs can occur. A warning sign is unexplained persistent fever with severe symptoms following exposure to COVID-19.

Affected children consistently show laboratory evidence of hyperinflammation. Pronounced biological markers of inflammation generally include strongly raised erythrocyte sedimentation rate, ESR, C-reactive protein, CRP, procalcitonin, ferritin, and IL-6. Low platelet counts and impaired blood clotting are also common, with increased levels of D-dimer and fibrinogen.

Clinical features can appear similar to Kawasaki disease, a rare disease that typically affects young children. Differences with respect to Kawasaki disease include frequent presentation with gastrointestinal symptoms such as vomiting, diarrhoea, and abdominal pain. Neurological involvement also appears to be relatively frequent. Characteristic laboratory findings that are not usually encountered in Kawasaki disease include very high levels of ventricular natriuretic peptide (a marker of heart failure), as well as somewhat lower platelet counts, lower absolute lymphocyte counts, and higher CRP levels.

The pathogenesis is not completely known and could implicate several factors. SARS-CoV-2 could have one of several roles: it could act as an environmental trigger for the condition either directly or indirectly by somehow paving the way for a different trigger.

Understanding the pathophysiology is a key research priority. Questions regarding the underlying molecular mechanisms that lead to the disorder following exposure to SARS-CoV-2 include identification of any genetic predisposition factors; any associations with particular viral variant/s; any molecular patterns capable of triggering the autoimmune/autoinflammatory responses. Another key question is whether the molecular mechanisms that trigger autoimmune/autoinflammatory responses in children with PIMS and adults with severe COVID-19 (including the induction of high concentrations of IL-6) are similar or distinct.

### Background to long COVID

Long COVID is a condition characterized by long-term consequences persisting or appearing after the typical convalescence period of COVID-19. It is an increasingly recognized problem facing the globally infected population and its health systems. Long COVID is also known as post-COVID-19 syndrome, post-COVID-19 condition, post-acute sequelae of COVID-19, PASC, or chronic COVID syndrome, CCS.

The phrase long COVID generally describes those persons with COVID-19 who experience symptoms for >28 days after diagnosis, whether laboratory confirmed or clinical. Symptoms are as markedly heterogeneous as seen in acute COVID-19 and may be constant, fluctuate, or appear and be replaced by symptoms relating to other systems with varying frequency. Although many persons with long COVID will be managed in primary care, others will require greater input from rehabilitation medicine experts.

Multiorgan symptoms after acute COVID-19 infections are being reported by increasing numbers of patients. They range from cough and shortness of breath, to fatigue, headache, palpitations, chest pain, joint pain, physical limitations, depression, and insomnia, and affect people of varying ages. Long COVID is a burgeoning health concern and action is needed now to address it.

Large and long-term cohort studies are urgently needed to help better understand the trajectory, complications, and biological mechanisms that drive the long-term health consequences of COVID-19. Although vaccination has become the immediate focus of the pandemic response for many countries, patients with long COVID must not be forgotten or sidelined as countries begin to consider the end of the pandemic.

Lawler N. G. et al. (2021) describes the detection of inflammation caused by an acute COVID-19 infection by determination of kynurenine as an inflammation marker in plasma and serum samples.

Thus, there exist an increasing need to understand the disease mechanisms, identify drug targets and inflammatory processes associated with a SARS-CoV-2 infection, especially in long COVID.

### Summary of the invention

The object of the present invention is to identify if kynurenine is able to depict the inflammatory situation during the acute phase of COVID-19. Furthermore, if kynurenine is also able to depict a subclinical inflammatory situation in patients with long COVID and/or PIMS.

The present invention discloses a method to detect and monitor acute COVID-19, long COVID and/or PIMS.

In a first aspect, the invention relates to an in vitro method for the detection of inflammation caused by an acute COVID-19 infection, long COVID and/or PIMS by determining the level of kynurenine in saliva.

In a second aspect, the invention relates to the use of kynurenine as a biomarker in the in vitro detection of inflammation caused by an acute COVID-19 infection, long COVID and/or PIMS, wherein the level of kynurenine in saliva is determined.

In a third aspect, the invention relates to the use of a test kit with which the level of kynurenine in saliva is determined by using the inventive method.

### Detailed description of the invention

The invention and embodiments thereof will be described below in further detail in connection with the drawings.

The present invention relates to the kynurenine pathway, KP. Tryptophan is an essential amino acid that can be metabolized through different pathways, a major route being the kynurenine pathway. This pathway is illustrated in Figure 1. The first enzyme of the pathway, indoleamine-2,3-dioxygenase, IDO-1, is strongly stimulated by inflammatory molecules, particularly interferon-y. Thus, the kynurenine pathway is often systematically up-regulated when the immune response is activated. The biological significance is that the depletion of tryptophan and generation of kynurenines play a key modulary role in the immune response. It was found surprisingly that the level of kynurenine measured in saliva can be used for the detection of inflammation caused by an acute COVID-19 infection, long COVID and/or PIMS.

As already mentioned above, IDO-1, an IFN-γ-inducible intracellular enzyme, catalyzes the first and rate-limiting step in the degradation of the essential amino acid tryptophan in the kynurenine pathway. The immunomodulatory effects of IDO-1 are represented by the prevention of T cell proliferation, promotion of T cell apoptosis, induction of T cell ignorance, energy, and generation of T regulatory cells.

The KP is the main metabolic route of tryptophan, TRP, degradation in mammals; it is responsible for more than 95% of the TRP catabolism in the human brain. The metabolites produced by this metabolic cascade, termed kynurenines, are involved in a number of physiological processes, including neurotransmission and immune responses. The KP also involves neurotoxic and neuroprotective metabolites, and alterations in their delicate balance have been demonstrated in multiple pathological processes.

The term "kynurenine(s)", as used herein, refers to all metabolites of the tryptophan/kynurenine pathway. Thus, kynurenine and/or its derivatives are included in said term.

It should be noted that the term "comprising" does not exclude other elements or steps and that the singular terms "a" or "an" does not exclude a plurality. Further, elements described in association with different embodiments may be combined.

It has also to be noted that aspects of the invention have been described with reference to different subject-matters. In particular, some aspects have been described with reference to method type claims, whereas other aspects have been described with reference to use type claims and/or product type claims, respectively. However, a person skilled in the art will gather from the above and the following description that, unless otherwise notified, in addition to any combination between features belonging to one type of subject-matter also any combination between features relating to different types of subject-matters is considered to be disclosed with this text. In particular, combinations between features relating to the method type claims and features relating to the use type claims and/or product type claims are considered to be disclosed.

The central intermediate of the KP is L-kynurenine, L-KYN, where the metabolic pathway divides into two different branches. L-KYN is transformed to either the neuroprotective kynurenic acid, KYNA, via kynurenine aminotransferase, KAT, or 3-hydroxy-L-kynurenine, 3-OH-KYN. 3-OH-KYN is further metabolized in a sequence of enzymatic steps to finally yield NAD⁺ (as shown in Figure 1).

The present invention provides an in vitro method for the detection of inflammation caused by an acute COVID-19 infection, long COVID and/or PIMS wherein the content of kynurenine in saliva is determined. The present invention also provides methods and means in the form of test kits for performing the diagnostic method. The tests can easily be performed by the individuals or patients on their own and at any given time at which such determination seems appropriate when saliva is used as the sample.

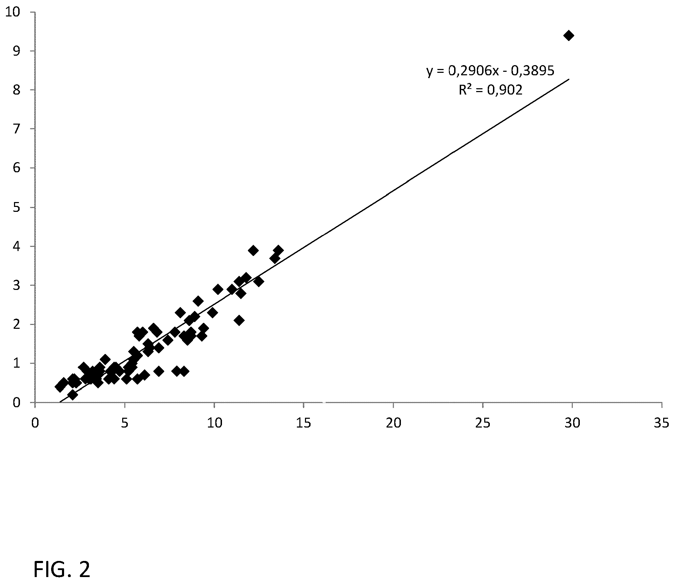

The inventive method is based on the surprising finding of the inventors that the kynurenine level in especially saliva proportionally corresponds very well with the kynurenine levels that have been determined in serum samples (Figure 2). Accordingly, the method of the present invention is performed with saliva.

In the inventive method, the level of kynurenine in saliva is determined. Whereas in the method of the present invention the level of L-kynurenine is preferably determined, it is, however, also possible to determine the level of N-formylkynurenine, 3-hydroxykynurenine and kynurenic acid.

Depending on the method of detection it may be possible to determine between the different intermediates. It is, however, also possible to use determination tests which react with the different intermediates. The determination of kynurenine is preferably performed quantitatively or semi-quantitatively since it is important to detect changes of the level of kynurenine which are outside the regular range. It is particularly advantageous that the in vitro method can be performed without a doctor or medically trained people when using saliva as a sample.

With the inventive method it is possible to determine a reliable relation between kynurenine levels in serum and saliva (Figure 2). There exists a linear correlation (R² = 0,902) between the measurement of kynurenine in serum and saliva. The relation is 3,3 (serum) : 1 (saliva). This observation proves that kynurenine is present at a significant and meaningful level in saliva, which is not the case for many other proteins.

Taking advantage of this observation, the present invention provides a reliable method and the use of a test kit for the detection of inflammation caused by an acute COVID-19 infection, long COVID and/or PIMS by determining the level of kynurenine in saliva.

The subject-matter of the invention is defined in the independent claims.

Preferred embodiments of the invention are defined in the respective dependent claims.

According to the invention, an in vitro method for the detection of inflammation caused by an acute COVID-19 infection, long COVID and/or PIMS is provided, wherein the level of kynurenine in saliva is determined and wherein the value of kynurenine measured in the patient to be diagnosed is compared with the average value obtained from a comparable cohort of persons who do not suffer from any of the diseases, whereby the value of kynurenine in patients is increased.

According to a preferred embodiment, the determination of kynurenine in saliva is used for therapy control.

According to another preferred embodiment, the determination of kynurenine in saliva is used for monitoring an acute COVID-19 infection, long COVID and/or PIMS and/or the recovery of a patient.

Further, according to a preferred embodiment, the level of kynurenine is at least two times higher in a patient than in the control group. More preferably, the level of kynurenine in a patient is at least 1 µM in saliva. Even more preferably, the level of kynurenine in saliva is 1,5 µM to 4 µM.

According to yet another preferred embodiment, an acute COVID-19 infection is a contagious disease caused by severe acute respiratory syndrome coronavirus 2, SARS-CoV-2.

In yet another preferred embodiment, long COVID is a condition characterized by long-term consequences persisting or appearing after the typical convalescence period of COVID-19.

According to still another preferred embodiment, PIMS is a systemic pediatric illness involving persistent fever and extreme inflammation following exposure to SARS-CoV-2.

According to the present invention, the method described herein is performed with saliva. Thus, it is possible to detect inflammation without the requirement of invasive measures. The patient can easily perform the tests by using his saliva, and the test kits provided herein allow an indication of potential risks in therapy.

According to still another preferred embodiment, the in vitro method is an ELISA test, or a lateral flow immunochromatographic assay, or a microfluidic test, or a colorimetric test, or an immunoblot.

The methods disclosed herein should be used together with clinical parameters. The relative value of kynurenine may preferably be interpreted together with other clinical parameters. The present invention contributes substantially to the prognostic value of the diagnosis. The method of the present invention is improved by comparing the value of kynurenine measured in the patient to be diagnosed with the average value obtained from a comparable cohort of persons who do not suffer from the disease.

In another embodiment, the present invention provides the use of kynurenine as a biomarker in the in vitro detection of inflammation caused by an acute COVID-19 infection, long COVID and/or PIMS, wherein the level of kynurenine in saliva is determined.

According to another preferred embodiment, the level of kynurenine as a biomarker for inflammation is at least 1 µM in saliva.

In another subject, the present invention provides the use of test kits with which the level of kynurenine in saliva is determined by using the inventive method. Such test kits comprise suitable means for performing the inventive method which may work on different principles. It is possible to use a specific color reagent which detects the presence of kynurenine and/or kynurenine derivatives. Alternatively, the kit may comprise at least one or preferably two antibodies specifically binding to kynurenine. Preferably when two antibodies are used, such antibodies do not bind to the same epitope in order to allow the formation of a sandwich formed by the first antibody, kynurenine or its derivatives and the second antibody.

According to a preferred embodiment, the test kit is an ELISA test kit, or a lateral flow immunochromatographic assay test kit, or a microfluidic test kit, or a colorimetric test kit or an immunoblot test kit.

The in vitro method as disclosed herein may be based on different principles. One of the preferred principles is known as Lateral Flow Immunochromatographic Assay. Such a Lateral Flow Immunochromatographic Assay can be easily performed by the patient without the help of a doctor or other medically trained person when saliva is used as the sample.

Lateral flow tests also known as Lateral Flow Immunochromatographic Assays are simple devices intended to detect the presence (or absence) of a target analyte sample without the need for specialized and costly equipment, though many lab based applications exist that are supported by a reading equipment. Typically, these tests are used for medical diagnostics either for home testing, point of care testing, or laboratory use. A widely spread and well-known application is the home pregnancy test. The technology is based on a series of capillary beds, such as pieces of porous paper or sintered polymer. Each of these elements has the capacity to transport fluid (e.g. saliva) spontaneously. The first element (the sample pad) acts as a sponge and holds an excess of sample fluid. Once soaked, the fluid migrates to the second element (conjugate pad) in which the manufacturer has stored the so called conjugate, a dried format of bio-active particles in a salt-sugar matrix that contains everything to guarantee an optimized chemical reaction between the target molecule and its chemical partner that has been immobilized on the particle's surface. While the sample fluid dissolves the salt-sugar matrix, it also dissolves the particles and in one combined transport action the sample and conjugate mix while flowing through the porous structure. In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripe-area changes color. Typically, there are at least two stripes: one (the control) that captures any particle and thereby shows that reaction conditions and technology worked fine, the second contains a specific capture molecule and only captures those particles onto which an analyte molecule has been immobilized. After passing these reaction zones the fluid enters the final porous material, the wick, that simply acts as a waste container. Lateral Flow Tests can operate as either competitive or sandwich assays.

In principle, any colored particle can be used, however, latex (blue color) or nanometer sized particles of gold (red color) are most commonly used. The gold particles are red in color due to localized surface plasmon resonance. Fluorescent or magnetic labeled particles can also be used, however these require the use of an electronic reader to assess the test result.

The sample first encounters colored particles which are labeled with antibodies raised to the target analyte. The test line will also contain antibodies to the same target, although it may bind to a different epitope on the analyte. The test line will show as a colored band in positive samples. While not strictly necessary, most test kits preferably incorporate a second line which contains an antibody that picks up free latex/gold in order to confirm the test has operated correctly.

In a preferred embodiment the single components of the lateral flow assay are adapted in such a manner that the presence of kynurenine is indicated only when more than a certain threshold value of kynurenine is present in the sample.

In another embodiment, the in vitro method of the present invention is performed as an ELISA, Enzyme Linked Immunosorbent Assay. There are different configurations of ELISA tests known. ELISA types are direct ELISA, sandwich ELISA, competitive ELISA and/or reverse ELISA.

Usually, a so-called sandwich ELISA is performed. In such an ELISA test, the compound that binds specifically to the analyte is fixed on a solid surface (e.g., the bottom of a microtiter well). Unspecific binding sites are saturated (e.g., with skim milk powder) in order to avoid unspecific binding. Usually, several microtiter wells are coated with the component in order to allow an easy dilution of the sample for a determination of the content of the analyte.

The binding of the analyte to the relevant wells is usually detected with another antibody that binds, however, to another area of the target molecule in order to avoid a negative interference of the binding. Such an antibody is usually coupled with a signal generating means that may be for example an enzyme like horseradish peroxidase. The presence of the analyte to be detected can then be seen by adding a precursor molecule, which is converted to another molecule having different properties by the signal generating molecule. When for example in one well kynurenine is present, the antibody binds to this molecule and with the activity of the signal generating means (e.g., horseradish peroxidase) a color signal is generated whereby the intensity is proportional to the amount of the bound target molecule. The reaction can be measured quantitatively and the amount of the analyte to be detected in the body fluid can be determined precisely.

A preferred test kit consists of the following components:
1. Sample pad - an absorbent pad onto the test sample is applied
2. Conjugate or reagent pad - this contains antibodies specific to the target analyte conjugated to colored particles (usually colloidal gold particles, or latex microspheres)
3. Reaction membrane - typically a hydrophobic nitrocellulose or cellulose acetate membrane onto which anti-target analyte antibodies are immobilized in a line across the membrane as a capture zone or test line (a control zone may also be present, containing antibodies specific for the conjugate antibodies)
4. Wick or waste reservoir - a further absorbent pad designed to draw the sample across the reaction membrane by capillary action and collect it.

The components of the strip are usually fixed to an inert backing material and may be presented in a simple dipstick format or within a plastic casing with a sample port and reaction window showing the capture and control zones.

In another embodiment of the present invention, the determination of kynurenine or its derivatives is performed by a coloring reaction. Before the content of kynurenine or its derivatives can be determined, components which may negatively affect the correct and precise test result have to be removed. Said undesired components of the sample which may disturb the correct test result are removed preferably by precipitation. Such precipitation can preferably be performed by using trichloric acid. It is, however, possible to use other methods for deproteinization of the components of the sample than using trichloric acid. After the disturbing components of the sample have been removed by precipitation it may be necessary to separate the phases by centrifugation. The supernatant is then preferably reacted with a coloring reagent which may preferably be Ehrlich's reagent. After development of the color, the samples are measured by measuring the absorbance at a suitable wavelength. Preferably, the test is performed in a quantitative or semi-quantitative manner. In the test method, either a calibration curve can be used or a certain threshold value is fixed in the test kit in order to avoid false positive results.

In still a further embodiment, the in vitro test method is a microfluidic test. Said test can be used to conduct an ELISA or LFA test on paper. The test comprises two parts: a sliding strip which contains the active sensing area, and a structure surrounding the sliding strip, which holds stored reagents like buffers, antibodies and enzymatic substrates, and distributes fluids. Running said test involves adding sample of a body fluid and water, moving the sliding strip at scheduled times, and analyzing the resulting color in the sensing area visually or using a flatbed scanner.

In yet another embodiment, the in vitro test method is an immunoblot. The basic principle of immunoblotting is the spatially separated fixation of defined antigens (blotting) on an easy-to-handle carrier matrix made of plastic or glass fiber, the application of a sample and the detection of the bound, specific antibodies for the corresponding antigen. The antigens are usually applied separately according to size. The oldest form of immunoblot is the Western blot, in which the antigens are previously separated electrophoretically and then transferred to a membrane. Also, dot blots and slot blots are performed as a kind of immunoblotting.

In practice, immunoblots have become established, in which defined antigen quantities are applied to a plastic strip by industrial printing processes, which can be used immediately in the laboratory for antibody detection. This type of immunoblot is also known as a line blot. The advantage of the line blot, in addition to the detection of specific antibody binding, is the control of the working steps by applied control antigens and the possible detection of a reaction against typically non-specific binding proteins. Antigens of completely different pathogens can also be present on the line blot (multiplex method), thus enabling simultaneous detection.

Another form of immunoblot is used in rapid test procedures (point-of-care testing, POCT), in which the antigens are fixed on an absorbent matrix and the sample (simultaneously also the detection reagents for antibody binding) is moved past the fixed antigen by capillary forces (immunochromatography ICT, lateral flow test, LFT or lateral flow assay, LFA).

### Description of the figures

**Figure 1** shows a schematic overview of the kynurenine pathway, the major route of the tryptophan degradation in higher eukaryotes. Enzymes are indicated in italics.
**Figure 2** shows a correlation of kynurenine concentrations measured in µM either in saliva (x-axis) or in serum (y-axis).
**Figure 3** shows the level of kynurenine in normal controls (n = 302) vs. COVID-19 patients in the early acute stage (1st week, n = 85). The difference was significant (p < 0.001).
**Figure 4** shows a comparison of different entities of infections with the disease of long COVID. There is a significant difference between infections (comprising pneumonia, urinary tract infections, and severe wound infections) and patients in the early phase of long COVID (p < 0.027). Patients after renal transplantation with CMV-reactivation/disease (n = 34) under immunosuppressive medication showed an increase of kynurenine levels too, but significantly lower compared to patients with long COVID.
**Figure 5** shows a measurement of kynurenine in serum and saliva in normal controls (n = 302) and in patients with an acute COVID-19 infection (n = 9). Both values (serum and saliva) in the COVID-19+ were significantly higher (p < 0.001).
**Figure 6** shows a comparison of kynurenine in serum in the follow up of Covid-19+ patients, either with (n = 9) or without (n = 28) an existing long COVID. Patients with the syndrome are currently under therapeutic management.
**Figure 7** shows a follow up of CRP-measurement in patients after COVID-19 infection and with long COVID. As can be seen, CRP was not a useful biomarker for follow-up in patients with long COVID.
**Figure 8** shows the level of kynurenine measured in serum in the 3rd month after positive PCR-testing: either cured or with a long-COVID syndrome for 3 months or more than 5 months. Kynurenine is still elevated significantly, whereas the values of the cured patients are in a normal range.

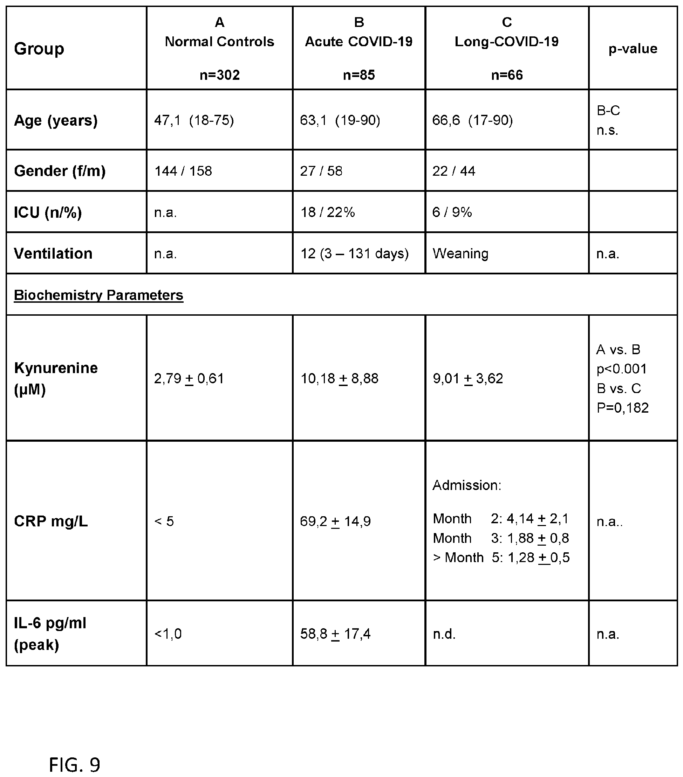

**Figure 9** shows the demographic and biochemical data of three different cohorts of patients (A, B and C) that were included in the study. Cohort A represents normal control patients without an infection by SARS-CoV-2. Cohort B represents long COVID-patients in the acute phase of the disease, either treated on the infection-ward or in the ICU. Cohort C represents patients under the diagnosis of long COVID disease. All three cohorts were comparable concerning age and gender distribution. There was a significant difference concerning kynurenine between the normal controls and patients with acute COVID-19 and long COVID.
   N = number of individuals tested
   n.s. = not significant
   n.a. = not available
   n.d. = not determined

The present invention is further illustrated by the following examples which are, however, not limiting the scope of the present invention.

### Example 1

Kynurenine test for the detection of inflammation caused by an acute COVID-19 infection, long COVID, and/or PIMS.

### 1.1 General used technique of colorimetric assay

The tryptophan metabolites via kynurenine can be quantitatively determined in biologic fluids by color reactions which are known since many decades. In general, a detection method via the formation of a colored reaction product can be performed by standard methods.

Microplate Readers are laboratory instruments designed to detect biological, chemical or physical events of samples in microtiter plates. They are widely used in research, drug discovery, bioassay validation, quality control as well as manufacturing processes in the pharmaceutical and biotechnological industry and academic organizations. Sample reactions can be assayed in 6-1536 well format microtiter plates. The most common microplate format used in academic research laboratories or clinical diagnostic laboratories is a 96-well (8 by 12 matrix) with a typical reaction volume between 100 and 200 µL per well.

Common detection modes for microplate assays are absorbance, fluorescence intensity, luminescence, time-resolved fluorescence, and fluorescence polarization. Absorbance detection has been available in microplate readers for more than 3 decades, and is used for assays such as ELISA assays, protein and nucleic acid quantification or enzyme activity assays. A light source illuminates the sample using a specific wavelength (selected by an optical filter, or a monochromator), and a light detector located on the other side of the well measures how much of the initial (100 %) light is transmitted through the sample: the amount of transmitted light will typically be related to the concentration of the molecule of interest.

### 1.2. Description of the test

This test was developed as a modified method.

Saliva withdrawn for PCR-Analysis for detection of COVID-19 antibodies (n = 9) of the patients was normally withdrawn every Monday, Wednesday and Friday between 7:00 and 8:00 o'clock a.m. After measurement of antibodies, the remaining saliva was stored at -30°C and served as a pool for longitudinal en bloc kynurenine measurements.

A color reagent was prepared and a dilution of a standard solution of kynurenine was also prepared. The color reaction is performed with a so-called "Ehrlich-Reagenz" which results in a yellow color. A solution comprising 2 % by weight dimethylaminobenzaldehyde dissolved in 20 % HCl is designated as "Ehrlich-Reagenz". Said coloring reagent serves for the detection of primary amino groups, pyrrole and indole derivatives as well. The colorimetric determination of the concentration is performed with monochromatic light. The standard solution of kynurenine was prepared by using L-kynurenine sulfate.

Equal amounts of sample were mixed with 100 µl trichloroacetic acid (30 %) thoroughly. After centrifugation the supernatant was measured. The absorbents of each sample at 492 nm were compared with the absorbents at 650 nm or 690 nm of the same sample. Then the absorbents of the controls (average of 5 wells) were subtracted from the absorbents of each well. By preparing a standard curve, the concentration of kynurenine in each sample could be determined.

### Example 2

Serum values of kynurenine were determined as follows:
Blood for routine monitoring of the patients was normally withdrawn every Monday, Wednesday and Friday between 7:00 and 8:00 o'clock a.m. After measurement of routine parameters the remaining serum was stored at -30°C and served as a pool for longitudinal en bloc kynurenine measurements.

The determination of the level of kynurenine in serum was performed as described under example 1.2.

As shown earlier, there exists a linear correlation between the measurement of kynurenine in serum and in saliva. The relation is 3,3 (serum) :1 (saliva) (Figure 2).

### Statistical analysis:

Descriptive data analysis and analysis of variance methods were used to characterize the data. All p-values are two-sided and considered to be descriptive. For a formal statement of descriptive significance a nominal type I error level of α = 0.05 (two-sided) was assumed.

The exact Mann-Whitney U test was performed for comparison of two groups with not normally distributed continuous variables.

### Results:

In a pilot-study, a cohort (A) of normal control patients (n = 302, range of age: 18 - 75 years, mean 47,1 y; gender 144 f/158 m), and a second cohort (B) of patients (n = 85, range of age: 19 - 90 years, mean 63,1 y; gender 27 f/58 m) treated either on the infection ward (n = 67) or the ICU (n = 18) were analyzed.

The third cohort (C) of patients (n = 66, range of age: 17 - 90 years, mean 66,6 y; gender 22 f/44 m) were treated either on the ICU (n = 6) or the normal ward (59). This group of patients was investigated mainly as part of a proof of concept to study long COVID. Patients were PCR negative and either under weaning at the ICU (n = 6) or the normal ward (n = 59).

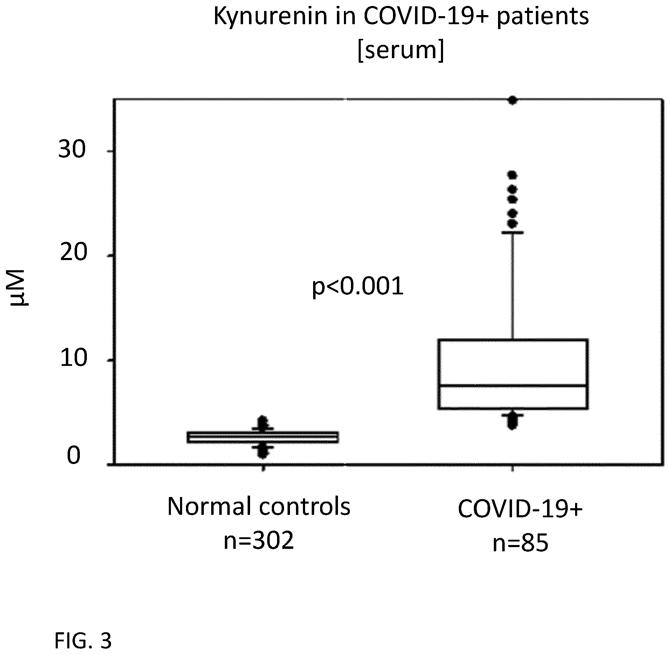

In a group of n = 85 patients infected with COVID-19, (mean age 52,8 ± 30 years) the inventors estimated kynurenine first in serum later also in saliva (n = 9). Kynurenine was significantly elevated in COVID-19 patients (cohort B, n = 85) compared to normal controls (cohort A, n = 302), (10,81±8,8 µM vs. 2,5±0,4 µM; p < 0,001) (Figure 3). Samples in cohort B were taken in the first week starting treatment. For better graphical demonstration, two results in the COVID-19+ group (58 and 43 µM), with a hyperinflammatory syndrome were left out.

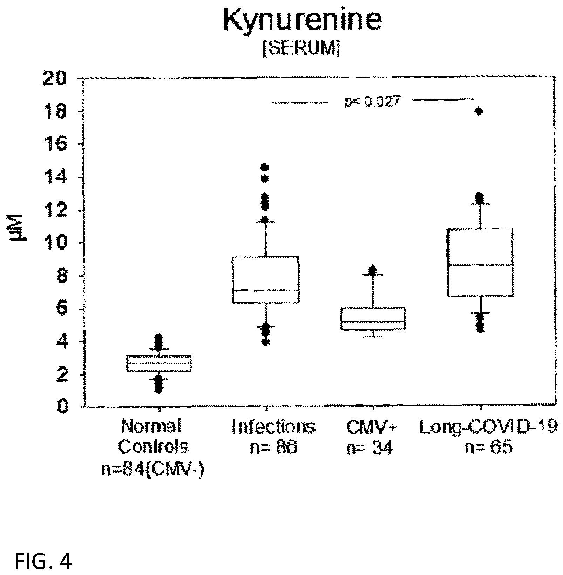

Compared to other types of infections, like cytomegalovirus, CMV, -reactivation / -disease or bacterial infections, there was a typical range of kynurenine elevation for each entity. All ranges were significantly different. Both types of virus infections, CMV and COVID-19, showed a significant rise of kynurenine levels, much more pronounced in the COVID-19 group. The picture of hyperinflammation with extraordinary high levels of kynurenine was solely found in COVID-19 patients (up to 57,91 µM, not shown for better graphical demonstration) (Figure 4).

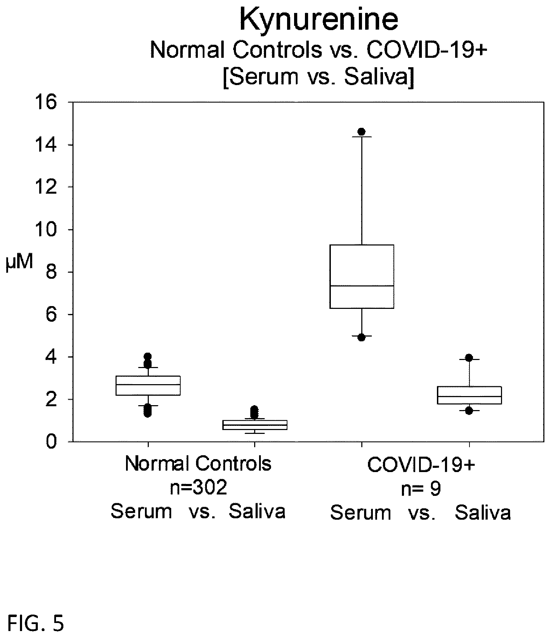

Kynurenine was measured not only in serum. In Figure 5, the inventors compared the measurements of kynurenine of normal controls and Covid-19+ patients (n = 9) either measured in serum or saliva. It could be shown that kynurenine levels in COVID-19+ patients are increased both in serum and saliva compared to levels of kynurenine in individuals without a SARS-CoV-2 infection (normal controls).

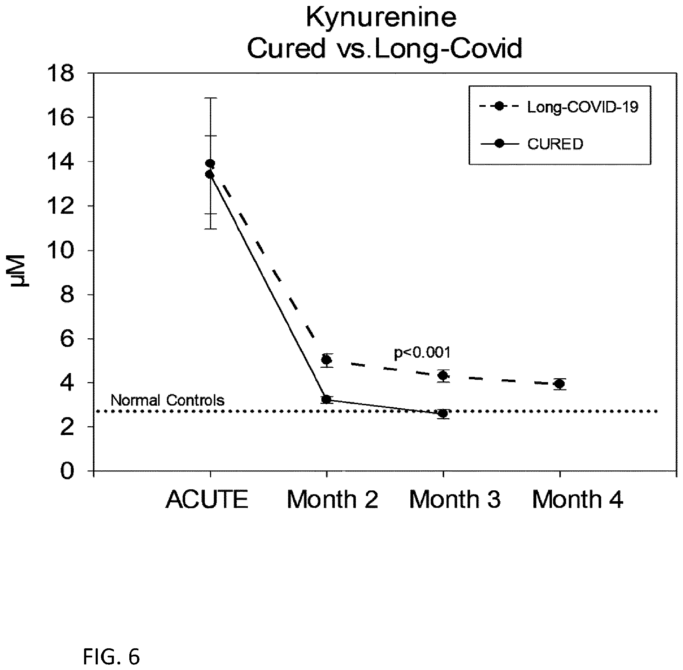

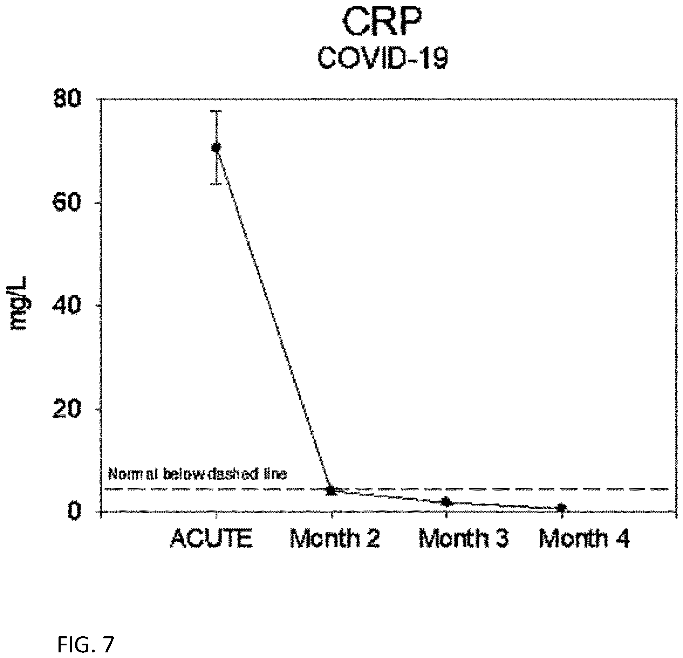

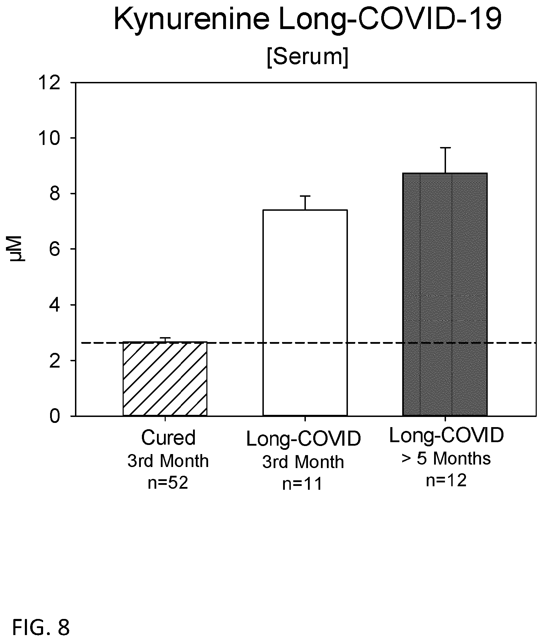

Looking in the follow-up of a starting group of patients (n = 9) with the diagnosis of long COVID, the inventors could demonstrate the sustained elevated level of kynurenine compared to normal controls from month 2 until month 4 (Figure 6). This was not found for CRP (Figure 7). 9 patients with signs of long COVID and 28 patients after a COVID-19+ infection without signs of long COVID were compared. Also, after more than 5 months, the level of kynurenine in long COVID patients was still elevated (Figure 8). CRP, described in the literature as a good biomarker in COVID-19 patients, was not useful in this study to identify long COVID. CRP at 2 months after positive testing with COVID-19 was near normal (normal range > 5 mg/L or 5 µg/ml) (Figure 7)

Kynurenine in COVID-19+ patients (n = 9) could be measured additionally in saliva and showed comparable results (Figure 5). Kynurenine levels of patients compromised by virus-infections (CMV vs. COVID-19) are comparable (Figure 4).

In total, it could be demonstrated by the present invention, that kynurenine is useful as a biomarker for conditions in COVID-19, thus, it is a useful biomarker in detecting inflammation caused by and monitoring of acute COVID-19, long COVID and/or PIMS. Kynurenine is able to depict the inflammatory and hyperinflammatory character of a SARS-CoV-2 disease. It was further shown by the present invention, that kynurenine is able to detect the chronic subclinical systemic inflammation seen in long COVID. This demonstrates also that kynurenine can be used for therapeutical monitoring.

Furthermore, measurements of kynurenine in saliva as shown in the present invention for the first time regarding an acute COVID-19 infection, long COVID and PIMS, open the opportunity for self-monitoring of the patient and a kind of therapy control.

## Claims

1. An in vitro method for the detection of inflammation caused by an acute COVID-19 infection, long COVID and/or pediatric inflammatory multisystem syndrome (PIMS), wherein the level of kynurenine in saliva is determined and wherein the value of kynurenine measured in the patient to be diagnosed is compared with the average value obtained from a comparable cohort of persons who do not suffer from said diseases, whereby the value of kynurenine in patients is increased.

2. In vitro method according to claim 1, wherein the determination of kynurenine in saliva is used for therapy control.

3. In vitro method according to claim 1, wherein the determination of kynurenine in saliva is used for monitoring acute COVID-19 infection, long COVID and/or PIMS and / or the recovery of a patient.

4. In vitro method according to any of the proceeding claims, wherein the level of kynurenine is at least two times higher in a patient than in the control group.

5. In vitro method according to any of the proceeding claims, **characterized in that** acute COVID-19 infection is a contagious disease caused by severe acute respiratory syndrome coronavirus 2 (SARS-CoV-2).

6. In vitro method according to any of the proceeding claims, **characterized in that** long COVID is a condition **characterized by** long-term consequences persisting or appearing after the typical convalescence period of COVID-19.

7. In vitro method according to any of the proceeding claims, **characterized in that** PIMS is a systemic paediatric illness involving persistent fever and extreme inflammation following exposure to SARS-CoV-2.

8. In vitro method according to any of the proceeding claims, **characterized in that** it is an ELISA test, or a lateral flow immunochromatographic assay, or a microfluidic test, or a colorimetric test, or an immunoblot.

9. Use of kynurenine as a biomarker in the in vitro detection of inflammation caused by an acute COVID-19 infection, long COVID and/or PIMS, wherein the level of kynurenine in saliva is determined.

10. Use of kynurenine according to claim 9, wherein the level of kynurenine is at least 1 µM in saliva.

11. Use of a test kit with which the level of kynurenine in saliva is determined by using the method according to any one of claims 1 to 8.

12. Use of a test kit according to claim 11, **characterized in that** it is an ELISA test kit, or a lateral flow immunochromatographic assay test kit, or a microfluidic test kit, or a colorimetric test kit, or an immunoblot test kit.

## Patentansprüche

1. In-vitro-Verfahren zum Nachweis einer Entzündung, die durch eine akute COVID-19-Infektion, Long COVID und/oder das pädiatrische inflammatorische Multisystem-Syndrom (PIMS) verursacht wird, wobei der Kynureninspiegel im Speichel bestimmt wird und wobei der bei dem zu diagnostizierenden Patienten gemessene Kynureninwert mit dem Durchschnittswert verglichen wird, der aus einer vergleichbaren Personengruppe gewonnen wurde, die nicht an den genannten Krankheiten leiden, wobei der Kynureninwert bei Patienten erhöht ist.

2. In-vitro-Verfahren nach Anspruch 1, wobei die Bestimmung von Kynurenin im Speichel zur Therapiekontrolle verwendet wird.

3. In-vitro-Verfahren nach Anspruch 1, wobei die Bestimmung von Kynurenin im Speichel zur Überwachung einer akuten COVID-19-Infektion, von Long COVID und/oder PIMS und/oder der Genesung eines Patienten verwendet wird.

4. In-vitro-Verfahren nach einem der vorhergehenden Ansprüche, wobei der Kynureninspiegel bei einem Patienten mindestens doppelt so hoch ist wie in der Kontrollgruppe.

5. In-vitro-Verfahren nach einem der vorhergehenden Ansprüche, **dadurch gekennzeichnet, dass** die akute COVID-19-Infektion eine ansteckende Krankheit ist, die durch das schwere akute respiratorische Syndrom Coronavirus 2 (SARS-CoV-2) verursacht wird.

6. In-vitro-Verfahren gemäß einem der vorhergehenden Ansprüche, **dadurch gekennzeichnet, dass** Long COVID ein Zustand ist, der durch langfristige Folgen gekennzeichnet ist, die nach der typischen Genesungsphase von COVID-19 bestehen bleiben oder auftreten.

7. In-vitro-Verfahren gemäß einem der vorhergehenden Ansprüche, **dadurch gekennzeichnet, dass** PIMS eine systemische pädiatrische Erkrankung ist, die mit anhaltendem Fieber und extremen Entzündungen nach Exposition mit SARS-CoV-2 einhergeht.

8. In-vitro-Verfahren gemäß einem der vorhergehenden Ansprüche, **dadurch gekennzeichnet, dass** es sich um einen ELISA-Test, einen immunochromatographischen Lateral-Flow-Assay, einen mikrofluidischen Test, einen kolorimetrischen Test oder einen Immunoblot handelt.

9. Verwendung von Kynurenin als Biomarker beim In-vitro-Nachweis einer Entzündung, die durch eine akute COVID-19-Infektion, Long COVID und/oder PIMS verursacht wird, wobei der Kynureninspiegel im Speichel bestimmt wird.

10. Verwendung von Kynurenin gemäß Anspruch 9, wobei der Kynureninspiegel im Speichel mindestens 1 µM beträgt.

11. Verwendung eines Testkits, mit dem der Kynureninspiegel im Speichel mittels eines Verfahrens gemäß einem der Ansprüche 1 bis 8 bestimmt wird.

12. Verwendung eines Testkits gemäß Anspruch 11, **dadurch gekennzeichnet, dass** es sich um ein ELISA-Testkit, ein immunochromatographisches Lateral-Flow-Testkit, ein mikrofluidisches Testkit, ein kolorimetrisches Testkit oder ein Immunoblot-Testkit handelt.

## Revendications

1. Procédé in vitro pour la détection d'une inflammation causée par une infection aiguë au COVID-19, un COVID long et/ou un syndrome inflammatoire pédiatrique multisystémique (PIMS), dans lequel le taux de kynurénine dans la salive est déterminé et dans lequel la valeur de kynurénine mesurée chez le patient à diagnostiquer est comparée à la valeur moyenne obtenue à partir d'un groupe comparable de personnes qui ne souffrent pas desdites maladies, la valeur de kynurénine chez les patients étant augmentée.

2. Procédé in vitro selon la revendication 1, dans lequel la détermination de la kynurénine dans la salive est utilisée pour le contrôle thérapeutique.

3. Procédé in vitro selon la revendication 1, dans lequel la détermination de la kynurénine dans la salive est utilisée pour surveiller une infection aiguë au COVID-19, un COVID long et/ou un PIMS et/ou le rétablissement d'un patient.

4. Procédé in vitro selon l'une des revendications précédentes, dans lequel le niveau de kynurénine est au moins deux fois plus élevé chez un patient que dans le groupe témoin.

5. Procédé in vitro selon l'une des revendications précédentes, **caractérisé en ce que** l'infection aiguë au COVID-19 est une maladie contagieuse causée par le syndrome respiratoire aigu sévère coronavirus 2 (SARS-CoV-2).

6. Procédé in vitro selon l'une des revendications précédentes, **caractérisé en ce que** le COVID long est une pathologie **caractérisée par** des conséquences à long terme qui persistent ou apparaissent après la période de convalescence typique du COVID-19.

7. Procédé in vitro selon l'une des revendications précédentes, **caractérisé en ce que** le PIMS est une maladie pédiatrique systémique impliquant une fièvre persistante et une inflammation extrême après une exposition au SARS-CoV-2.

8. Procédé in vitro selon l'une des revendications précédentes, **caractérisé en ce qu'**il s'agit d'un test ELISA, d'un test immunochromatographique à flux latéral, d'un test microfluidique, d'un test colorimétrique, ou d'un immunoblot.

9. Utilisation de la kynurénine comme biomarqueur dans la détection in vitro de l'inflammation causée par une infection aiguë au COVID-19, un COVID long et/ou un PIMS, dans laquelle le niveau de kynurénine dans la salive est déterminé.

10. Utilisation de la kynurénine selon la revendication 9, dans laquelle le taux de kynurénine est d'au moins 1 µM dans la salive.

11. Utilisation d'un kit de test permettant de déterminer le taux de kynurénine dans la salive à l'aide du procédé selon l'une des revendications 1 à 8.

12. Utilisation d'un kit de test selon la revendication 11, **caractérisée en ce qu'**il s'agit d'un kit de test ELISA, d'un kit de test immunochromatographique à flux latéral, d'un kit de test microfluidique, d'un kit de test colorimétrique, ou d'un kit de test immunoblot.
